# EUROPÄISCHE PATENTSCHRIFT

(11) **EP 3 595 607 B1**
(45) Veröffentlichungstag und Bekanntmachung des Hinweises auf die Patenterteilung: **18.11.2020**
(21) Anmeldenummer: 18725509.6
(22) Anmeldetag: 17.05.2018
(51) Int. Cl.: A61G 13/00, A61G 13/12, A61G 13/10

(54) **CHIRURGISCHES ROTATIONSAGGREGAT MIT VERBESSERTER ROTATIONSVERRIEGELUNG**
SURGICAL ROTATIONAL UNIT WITH IMPROVED ROTATIONAL LOCKING
ENSEMBLE CHIRURGICAL ROTATIF DOTÉ D'UN VERROUILLAGE DE ROTATION AMÉLIORÉ

(30) Priorität: 17.05.2017 DE 102017110719
(43) Veröffentlichungstag der Anmeldung: 22.01.2020
(73) Patentinhaber: Maquet GmbH, 76437 Rastatt (DE)
(72) Erfinder: KOCH, Guido, 76149 Karlsruhe (DE)
(74) Vertreter: Zacco GmbH
(86) Internationale Anmeldenummer: PCT/EP2018/062944
(87) Internationale Veröffentlichungsnummer: WO 2018/211017

(56) Entgegenhaltungen:
- DE-A1-102014 108 745
- US-A1- 2013 191 994
- US-A1- 2016 151 224

## Beschreibung

### Gebiet der Erfindung

Die vorliegende Offenbarung betrifft Operationstische und deren Zubehör, einschließlich solche(s) auf dem Gebiet der orthopädischen Chirurgie, insbesondere der orthopädischen Extension.

### Beschreibung des Standes der Technik

Die vorliegende Offenbarung betrifft allgemein chirurgische Rotationsaggregate. Ein Rotationsaggregat in der Form eines Zugspindelaggregats 210 ist aus der von Malcolm Wootton eingereichten internationalen Patentanmeldung WO 2013/034916 A1 bekannt, siehe hierzu die Figur 18 und Seite 22 der Beschreibung. Solche Zugspindelaggregate werden bei bestimmten chirurgischen Eingriffen eingesetzt, wie es im Folgenden beschrieben wird.

Bei der Operation von Patienten ist es oft erforderlich, dass Teile des Patienten, insbesondere Gliedmaßen fixiert und gezielt bewegt werden müssen. Insbesondere bei Operationen, bei denen die Lage eines Körperteils mehrfach gezielt geändert werden muss, sind einstellbare Behandlungsvorrichtungen erforderlich. So muss bei orthopädischen Eingriffen, wie z.B. einer hüftprothetischen Prozedur nach dem "direct anterior approach" sowie bei der "total hip arthroplasty", das Patientenbein frei zugänglich gelagert und wiederholt rotiert werden.

Das Patientenbein ist hierbei in einer Traktions- bzw. Zugeinheit freitragend gerade eingespannt. Solche Traktions- bzw. Zugeinheiten werden in Verbindung mit Operationstischen auch als Extensionsvorrichtungen bezeichnet. Diese Extensionsvorrichtungen umfassen mindestens einen Extensionsholm, der an seinem einen Ende über eine Schnittstelle mit dem Operationstisch verbindbar ist und an seinem anderen Ende eine Halterung für die Zugeinheit hat. Der Extensionsholm kann mit einer Patientenlagerfläche des Operationstischs verbunden oder an dieser befestigt sein.

Dazu wird der Fuß des Patienten in einem sogenannten Extensionsschuh aufgenommen, an dessen Sohle ein Zugspindelaggregat, wie z.B. das von Wootton beschriebene, adaptiert ist. Über dieses Zugspindelaggregat kann in der Längsrichtung des Beins eine Zugkraft aufgebracht werden, um beispielsweise bei der Reposition die Bruchkanten des Knochens in ihre ursprüngliche Position zu bringen. Zusätzlich soll das Bein um seine Längsachse nach innen und nach außen schwenkbar sein. Hierfür muss der Rotationswinkel des Beins entsprechend dem chirurgischen Workflow und den medizinischen Notwendigkeiten mehrfach geändert werden. Der maximale übliche Rotationsbereich beträgt ±180°.

Zum Ändern des Rotationswinkels eines über das Zugspindelaggregat von Wootton eingespannten Patientenbeins muss ein Bediener einen drehbaren Rotor, der in einem als Klemmschelle ausgebildeten Stator aufgenommenen ist, mittels eines Handgriffs 269 manipulieren. Dabei muss der Bediener zur Freigabe der Rotation mit einer Hand einen Klemmhebel 268 umlegen, sodass die Rotation des Patientenbeins durch die Extensionsvorrichtung freigegeben wird. Anschließend dreht der Bediener das Patientenbein um dessen Längsachse in die gewünschte Position. Dabei kann die Fußspitze des Patienten als Indikator für den Drehwinkel um die Längsachse genutzt werden. Das Patientenbein muss dann im gewünschten Rotationswinkel einhändig manuell gehalten werden, während eine Rückstellkraft in Gegenrichtung zur durchgeführten Drehung wirkt. Zum Arretieren wird der Klemmhebel 268 anschließend mit der anderen Hand angezogen, sodass das Patientenbein von der Extensionsvorrichtung in dem gewünschten Rotationswinkel fixiert wird. Um das Patientenbein in einen anderen Rotationswinkel zu bringen, muss der Bediener den Handgriff 269 unter einer hohen Lastanwendung mit einer Hand loslassen, um mit der freien Hand den Klemmhebel 268 wieder umzulegen, und das Patientenbein erneut kontrolliert in die gewünschte Lage zu drehen. Anschließend wird der Klemmhebel 268 wieder einhändig fixiert.

Aus der Praxis ist es bekannt, dass diese Prozedur während einer Operation mehrfach wiederholt werden muss. Im erläuterten Ablauf sind die Tätigkeiten Lösen/Festziehen des Klemmhebels 268 und Rotieren/Halten des Patientenbeins in der gewünschten Position teilweise gleichzeitig durchzuführen. Das Rückstellbestreben des Patientenbeins in eine definierte Lage ist dabei oft erschwerend und führt zu einer erheblichen Belastung des Bedieners, da erhebliche Handkraft und Konzentration zur einhändigen Koordination der erforderlichen Arbeitsschritte benötigt werden.

Eine feinfühlige Positionierung des Patientenbeins ist somit während einer Operation nur eingeschränkt möglich, sodass hierdurch ein Risiko für eine ungewollte Verstellung des Winkels während der Rotation des Patientenbeins oder bei einer ungenügenden Fixierung des Klemmhebels 268 für den Patienten gegeben ist. Um dieses Risiko zu minimieren, ist es üblich, dass eine Person den Klemmhebel 268 umlegt, während eine zweite Person das Patientenbein in die gewünschte Lage bringt und dort hält, bis der Klemmhebel 268 wieder festgestellt ist. Mit zwei Personen sind der Aufwand und die anfallenden Kosten hierfür relativ hoch. Ferner steigt bei der Prozedur die Kontaminationsgefahr.

Aus dem Dokument DE 10 2014 108 745 A1 ist ein weiteres Zugspindelaggregat bekannt. Bei diesem gibt es eine Verriegelungsanordnung, die dadurch betätigt wird, dass der vorgesehene Drehgriff in eine der beiden Drehrichtungen rotiert wird.

Das Dokument DE 10 2012 112 716 A1 beschreibt einen medizinischen Haltearm umfassend mindestens ein Gelenk mit zwei Gelenkkörpern, die um eine Drehachse gegeneinander verdrehbar sind, wobei ein erster der Gelenkkörper mehrere Raststifte und ein zweiter der Gelenkkörper mehrere Rastvertiefungen aufweist, und wobei die Zahl an Raststiften verschieden von der Zahl an Rastvertiefungen ist.

### Zusammenfassung der Offenbarung

Die vorliegende Offenbarung betrifft unter anderem Rotationsaggregate ("rotation assemblies"), Extensionsholme mit Rotationsaggregaten, Operationstische mit Rotationsaggregaten, Operationstische mit Extensionsholmen samt Rotationsaggregaten, sowie Verfahren zur Verwendung von Rotationsaggregaten bei chirurgischen Eingriffen.

Eine Aufgabe der vorliegenden Offenbarung ist es, ein Rotationsaggregat bereitzustellen, bei dem das Rotieren und Arretieren eines Patientenbeins sogar unter hohen Lastanwendungen sicher und bequem durch einen Benutzer gehandhabt werden kann.

Diese Aufgabe wurde gelöst durch ein Rotationsaggregat nach Anspruch 1, und betrifft ein Rotationsaggregat zur Anbindung an einen Operationstisch zwecks Drehung eines Patientenbeins um seine Längsachse, wobei das Rotationsaggregat alle oder einen Teil der folgenden Merkmale hat:
- eine Schnittstelle zur Befestigung des Fußes des Patientenbeins am Rotationsaggregat;
- einen Rotor, mit dem die Schnittstelle drehfest verbunden ist und der zur Drehung der Schnittstelle und damit des Patientenbeins um die Längsachse dient;
- einen Stator, an dem der Rotor drehbar gelagert ist, sodass der Rotor sich relativ zum Stator drehen kann;
- einen Drehgriff zur Drehung des Rotors; und
- eine Verriegelungsanordnung zur Sperrung der Drehung des Rotors relativ zum Stator,
wobei der Drehgriff verschieblich auf dem Rotor gelagert ist, und
wobei die Verriegelungsanordnung durch Verschiebung des Drehgriffs auf dem Rotor betätigt wird.

Dadurch, dass die Verriegelungsanordnung durch Einwirkung auf den Drehgriff betätigt werden kann, kann der Benutzer die Verriegelungsanordnung lösen, das Patientenbein verdrehen, und die Verriegelungsanordnung wieder feststellen, ohne dass er die Hände vom Drehgriff nehmen muss. Während des ganzen Vorgangs zur Drehung des Patientenbeins kann der Benutzer beide Hände am Drehgriff belassen und ohne fremde Hilfe die Verriegelungsanordnung bedienen. Dadurch lässt sich das Rotieren und Arretieren des Patientenbeins bequem durch eine einzige Person bewerkstelligen.

Der Drehgriff dient dem Einstellen eines gewünschten Drehwinkels und dem Betätigen der Verriegelungsanordnung. Gemäß einigen Ausführungsformen kann der Drehgriff mit dem Rotor drehfest gekoppelt sein.

Die Verriegelungsanordnung kann mindestens zwei Gruppen von Raststiften und eine Mehrzahl von Rastvertiefungen umfassen, wobei die Anzahl der Raststifte verschieden von der Anzahl der Rastvertiefungen ist. Die mindestens zwei Gruppen von Raststiften und eine Mehrzahl von Rastvertiefungen können am Umfang der Verriegelungsanordnung verteilt sein. Die Rastvertiefungen können bei einem Verstellbereich von ± 180° um den kompletten Umfang des Stators gleichmäßig verteilt sein. Durch die Verriegelungsanordnung mit mindestens zwei Gruppen von Raststiften und einer davon verschiedenen Mehrzahl von Rastvertiefungen kann das Freigeben und Arretieren einer Drehbewegung des Rotors auch unter hohen Lastanwendungen erfolgen. Diese Verriegelungsanordnung kann beispielsweise zur Sperrung der Rotation einer über das Rotationsaggregat eingespannten Extremität angewendet werden. Bei anderen Operationstechniken kann sogar eine freie Rotation unter bedienerfreundlichen Bedingungen stattfinden. Weitere medizinische und nichtmedizinische Anwendungsgebiete sollten nicht ausgeschlossen werden.

Des Weiteren können die am Stator ausgebildeten Rastvertiefungen der vorliegenden Verriegelungsanordnung eine im Voraus festgelegte Winkelteilung aufweisen. Dabei unterscheidet sich die Winkelteilung der Raststifte der jeweiligen Gruppe der mindestens zwei Gruppen von Raststiften von der Winkelteilung der Rastvertiefungen um einen Differenzwinkel "α". Die Anzahl der Raststifte ist dabei so bemessen, dass der Winkelsektor der Raststifte ein geradzahliges Vielfaches der Winkelteilung der Rastvertiefungen ergibt. So erhält man für die Verriegelungsanordnung eine Vielzahl von über 360° verteilten Einrast- oder Verriegelungspositionen. Bei jeder Verriegelungsposition steht ein Raststift der jeweiligen Gruppe der mindestens zwei Gruppen von Raststiften einer Rastvertiefung gegenüber. Der Winkelabstand zwischen zwei Verriegelungspositionen ist dem Differenzwinkel "α" gleich.

Gemäß einer Ausführungsform kann die Drehbewegung des Rotors gegenüber dem Stator arretiert werden, wenn keine axiale Betätigungskraft zwecks Entriegelung auf den Drehgriff ausgeübt wird. Hierfür tritt jeweils einer der Raststifte aus jeder Gruppe der mindestens zwei Gruppen von Raststiften, der einer Rastvertiefung exakt gegenüberliegt, mit seinem einen Ende unmittelbar in diese Vertiefung ein. Sind die mindestens zwei Gruppen von Raststiften in Winkelabständen angeordnet, die ein Vielfaches der Winkelteilung der Rastvertiefungen betragen, so rastet jeweils einer der Raststifte der jeweiligen Gruppe der mindestens zwei Gruppen von Raststiften gleichzeitig ein. Sämtliche weiteren Raststifte der mindestens zwei Gruppen von Raststiften, die auf keine Rastvertiefung treffen, können nicht einrasten. Besonders positiv wirkt sich das Verriegeln mit gleichzeitig mehreren Raststiften, die jeweils einer anderen Gruppe von Raststiften zugehören, auf die Belastungsfähigkeit und Stabilität der Verriegelungsanordnung bei hohen Drehmomenten aus. Die Verriegelungsanordnung gewährleistet ein sicheres Sperren der Drehung des Rotors.

Ferner ermöglicht die vorliegende Verriegelungsanordnung eine Freigabe der Drehbewegung des Rotors. Die Drehung des Rotors kann durch eine gleichzeitige Entriegelung aller Raststifte freigegeben werden. Das Entriegeln kann durch das axiale Verschieben des Drehgriffs durchgeführt werden. Beispielsweise kann der Drehgriff dabei in eine dem Patienten abgewandte Richtung zurückgezogen werden, so dass dem Bediener die Möglichkeit eingeräumt wird, das Patientenbein zu verdrehen.

Die vorliegende Verriegelungsanordnung sorgt für eine verbesserte Bedienergonomie. Insbesondere kann dabei auf separat zu betätigende Klemm- oder Feststellelemente verzichtet werden. Somit kann eine exakte Positionierung des Rotors und eine Einstellung des gewünschten Drehwinkels gleichzeitig mittels eines Drehgriffs sichergestellt werden.

Die einfache Bauweise und eine bequeme Bedienung der Verriegelungsanordnung ermöglichen dem Bediener das Verstellen, Arretieren und Freigeben des Rotors ohne dabei den Drehgriff freizulassen und auf fremde Hilfe angewiesen zu sein.

Gemäß einigen Ausführungsformen können die Raststifte der mindestens zwei Gruppen von Raststiften am inneren Umfang des Rotors verteilt sein. Der Rotor kann beispielsweise am inneren Umfang mehrere Durchgangsbohrungen oder andere Arten von Aussparungen zur Aufnahme der Raststifte aufweisen. Die Raststifte können in den jeweiligen Ausnehmungen axial ausgerichtet vorliegen. Eine radiale Ausrichtung der Raststifte in den jeweiligen Aussparungen ist jedoch auch möglich. Diese Anordnung der mindestens zwei Gruppen von Raststiften sorgt für eine erhöhte Sicherheit und Belastbarkeit der Verriegelungsanordnung in einem verriegelten Zustand des Rotors. Zusätzlich können die funktionellen Bauelemente der Verriegelungsanordnung, wie beispielswiese die Raststifte und Elemente, die Rastvertiefungen aufweisen, ausgetauscht werden. Dadurch wird eine erneute Ausrichtung der Raststifte ermöglicht.

Die Raststifte der mindestens zwei Gruppen von Raststiften können im Rotor federnd gelagert und in Richtung auf die Rastvertiefungen vorgespannt sein. Die Federvorspannung erhöht zusätzlich die Stabilität der Verriegelungsanordnung. Ferner kann die Federvorspannung zwecks einer zuverlässigen und vollständigen Einrastung der jeweiligen Raststifte stufenlos einstellbar sein.

Gemäß einer Ausführungsform können die mindestens zwei Gruppen von Raststiften und die Rastvertiefungen jeweils mindestens eine kreisförmige Anordnung bilden. Dies bedeutet, dass die Raststifte und die korrespondierenden Rastvertiefungen im Querschnitt jeweils entlang mindestens einer imaginären Kreislinie konzentrisch um die Drehachse des Rotationsaggregats angeordnet sein können.

In einigen Ausführungsformen können die mindestens zwei Gruppen von Raststiften und die Rastvertiefungen radial symmetrisch angeordnet sein. Die jeweiligen Anordnungen können beispielsweise radial und/oder axial verlaufen. Ferner können die Anordnungen der mindestens zwei Gruppen von Raststiften und der Rastvertiefungen den gleichen Radius aufweisen. In manchen Anordnungen können die mindestens zwei Gruppen von Raststiften und die Rastvertiefungen gleichmäßig über den Umfang des Rotors bzw. Stators verteilt sein.

Zum Verstellen eines in einer bestimmten Position festgestellten Rotors muss die Arretierung gelöst werden. Das Entriegeln der Verriegelungsanordnung kann durch ein vollständiges Herausziehen der Raststifte aus den gegenüberliegenden Rastvertiefungen erreicht werden. Dabei können beispielsweise die jeweiligen eingerasteten Raststifte der mindestens zwei Gruppen von Raststiften gleichzeitig gegen die ausgeübte Federvorspannung gezogen werden. Durch diesen Entriegelungsmechanismus kann der Rotor schnell ohne großen Aufwand neu positioniert werden. Das Entriegeln kann beispielsweise durch das Verschieben des Drehgriffs entlang der Drehachse des Rotationsaggregats erfolgen. In einer der Ausführungsformen kann der Drehgriff als ein Sterngriff ("star-shaped handle") vorliegen. Diese Ausgestaltung ist jedoch nur beispielhaft zu verstehen. So kann der Griff auch eine andere Form aufweisen, z.B. die mindestens eines Bedienhebels.

Ferner kann der Drehgriff an einer Schiebehülse angebracht sein und mit ihr zusammen verschoben werden. Die Schiebehülse kann zusätzlich eine Winkelskala umfassen, die eine exakte Positionierung des Patientenfußes vereinfacht. Die Schiebehülse kann im arretierten Zustand der Vorrichtung mit Federdruck gegen den Stator gepresst und dem Rotor gegenüber drehfest angeordnet vorliegen. Dies kann mit Hilfe von mindestens zwei Kugelreihen erreicht werden, die in entsprechenden axialen Bahnen zwischen dem Rotor und der Schiebehülse angeordnet sind, in axialer Richtung abrollen und in radialer Richtung keine Bewegung zulassen.

Die Raststifte können derart ausgebildet sein, dass deren Eingriffsteile während einer Drehbewegung sanft in die jeweiligen Rastvertiefungen einfinden und somit nur eine geringe Reibung verursachen. Dabei können die jeweiligen Endabschnitte der Raststifte ungleichmäßig, beispielsweise abgeflacht, rund, elliptisch, ballig oder kegelförmig, ausgebildet sein und dabei diverse Unregelmäßigkeiten, wie Ausbuchtungen oder Aussparungen, aufweisen.

Die eingerasteten Raststifte können mindestens zwei Abschnitte aufweisen. In einem verriegelten Zustand der Verriegelungsanordnung befindet sich dann ein erster Abschnitt eines eingerasteten Raststifts in einer am Stator ausgebildeten korrespondierenden Rastvertiefung, wobei ein zweiter Abschnitt des eingerasteten Raststifts radial über die jeweilige Rastvertiefung hervorsteht.

Die Schiebehülse kann am statorseitigen Ende ein Freigabeelement in Form eines Bunds aufweisen, der in den Bereich der Stifte hineinragt, aber in Ruhestellung keinen Kontakt zu den Stiften hat. Gemäß einigen Ausführungsformen kann das Freigabeelement als ein Freigabering ausgebildet sein, der derart ausgebildet ist, dass er beim Verschieben des Drehgriffs entlang der Drehachse die Raststifte der mindestens zwei Gruppen von Raststiften kontaktiert. Das Freigabeelement kann dabei jeweils den zweiten hervorstehenden Abschnitt der jeweiligen Raststifte kontaktieren. Ferner kann das Freigabeelement in der Form eines Rohrs, einer Manschette oder als ein unregelmäßiger Vorsprung vorliegen.

Das vorliegende Rotationsaggregat kann derart ausgebildet sein, dass der Rotor drehbar, aber axial nicht verschieblich im Stator gelagert vorliegt. Dabei kann der Rotor einen Auszug umschließen, der relativ zum Rotor axial verschieblich und rotationsfest ausgebildet ist. Dies wird durch zwei zylindrische Stifte am Patienten zugewandten Ende des Rotors erreicht, welche in korrespondierende Aussparungen am Auszug eingreifen. Der Auszug kann demzufolge entlang der Zylinderstifte gleiten, während sich das zu übertragende Drehmoment an diesen abstützt. Da die hart beschichteten Führungsflächen des Auszugs freiliegen, ist dieser mittels Gleitlagern im Rotor positioniert, die für Trockenlauf geeignet sind. Bei manchen Ausführungsformen sind die Zylinderstifte aufgrund hoher Kantenpressung auf der Oberfläche des Auszugs aus Kunststoff ausgebildet.

Das vorliegende Rotationsaggregat kann derart ausgebildet sein, dass die Verriegelungsanordnung in einer Freigabeposition feststellbar ist. Dabei kann der Drehgriff einen Knopf aufweisen, der dem Feststellen des Rotors in einer Freigabeposition dient. Das Feststellen des Rotors in einer Freigabeposition resultiert in der freien Rotierbarkeit des Rotationsaggregats. Dabei kommen auch andere Mechanismen in Betracht, wie zum Beispiel am Stator angebrachte Drückknöpfe, Arretierungspins oder Schalter, sofern sie geeignet sind, für die gewünschte Freigabe zu sorgen. Der Knopf kann betätigt werden, ohne dass der Drehgriff dabei losgelassen werden muss. Die Betätigung des Knopfes kann auch ferngesteuert erfolgen.

Die vorliegende Verriegelungsanordnung kann mittels des betätigbaren Knopfes dauerhaft gelöst werden, um dem Bediener die Möglichkeit zu geben, das Bein des Patienten frei zu manipulieren. Beispielsweise kann der Bediener hierfür die Schiebehülse wie zuvor beschrieben mittels des Drehgriffs zu sich heranziehen, dann den Arretierungsknopf hineindrücken und den Zug aufgeben. Der Knopf bleibt somit in der gedrückten Position und verhindert, dass die Schiebehülse in die verriegelte Position zurückfällt. Um die Rotation wieder zu verriegeln, wird erneut Zug auf die Schiebehülse aufgebracht. Somit wird der Knopf gelöst und der Rotor arretiert.

Die Verriegelungsanordnung kann derart ausgebildet sein, dass die Anzahl und Teilung der benachbarten Raststifte der mindestens zwei Gruppen von Raststiften und die Anzahl der korrespondierenden Rastvertiefungen verschieden ist. Das jeweilige Verhältnis der Raststifte zu den Rastvertiefungen der Verriegelungsanordnung ergibt sich aus der im Vorfeld gewählten Winkeldifferenz. Daraus folgend kann die Anzahl an Raststiften der mindestens zwei Gruppen von Raststiften größer als die Anzahl an Rastvertiefungen sein. Gemäß weiteren Ausführungsformen kann die Anzahl an Rastvertiefungen größer als die Anzahl an Raststiften der mindestens zwei Gruppen von Raststiften sein.

Die Verriegelungsanordnung kann derart ausgebildet sein, dass der Rotor mindestens zwei Gruppen von Raststiften umfasst. Zum Arretieren der Drehbewegung des Rotors kann jeweils einer der Raststifte der mindestens zwei Gruppen von Raststiften mit einem Abschnitt vollständig in jeweils einer der Rastvertiefungen aufgenommen sein. Beispielsweise kann der Rotor vier Gruppen von Raststiften umfassen, wobei zum Arretieren der Drehbewegung des Rotors jeweils einer der Raststifte der vier Gruppen von Raststiften mit einem Abschnitt vollständig in jeweils einer der Rastvertiefungen aufgenommen ist.

Je nach Ausführung der Verriegelungsanordnung können auch die Anzahl der Raststifte innerhalb der mindestens zwei Gruppen von Raststiften und Rastvertiefungen variieren. Die Verriegelungsanordnung kann beispielsweise am Umfang des Rotors vier Gruppen von Raststiften mit jeweils sechs Raststiften pro Gruppe aufweisen, wobei die regelmäßige Winkelteilung der Raststifte 12° beträgt. Ferner umfasst diese Ausführungsform 25 korrespondierende Rastvertiefungen an der Anlagefläche des Stators, die kreisförmig am vollen Umfang im Abstand von 14,4° verteilt sind. Da die vier Gruppen von Raststiften jeweils um ein Vielfaches von 14,4° zu einander beabstandet sind, rastet jeweils ein Raststift aus jeder Gruppe der Raststiften beim Arretieren gleichzeitig ein. Ferner kann bereits bei einer Rotation von 2,4° der nächste Satz von Raststiften einrasten, dies entspricht der Differenz der beiden Teilungen. Dadurch wird eine feine, fast stufenlose Rastierung erzeugt, die wesentlich kleiner ist als die Teilung der Raststifte.

### Kurze Beschreibung der Zeichnungen

Beispielhafte Ausführungsformen der vorliegenden Offenbarung werden nachstehend unter Bezugnahme auf die angehängten Zeichnungen beschrieben, in welchen gleiche Bezugszeichen jeweils gleiche oder einander entsprechende Elemente bezeichnen.
- Fig. 1: zeigt eine Darstellung eines Operationstisches mit einem Extensionsholm, bei dem ein Rotationsaggregat in Form eines Zugspindelaggregats mit einer Verriegelungsanordnung gemäß einer Ausführungsform der vorliegenden Offenbarung eingesetzt werden kann.
- Fig. 2: zeigt ein Zugspindelaggregat mit einer Verriegelungsanordnung gemäß einer Ausführungsform der vorliegenden Offenbarung.
- Fig. 3: ist ein Längsschnitt durch den hinteren Teil des Zugspindelaggregats aus Fig. 2.
- Fig. 4: zeigt einige Bauteile der Verriegelungsanordnung des Zugspindelaggregats aus Fig. 2.
- Fig. 5: zeigt Raststifte und Rastvertiefungen der Verriegelungsanordnung des Zugspindelaggregats aus Fig. 2.
- Fig. 6: zeigt weitere Bauteile der Verriegelungsanordnung des Zugspindelaggregats aus Fig. 2.
- Fig. 7: zeigt ein Detail des Zugspindelaggregats aus Fig. 2.
- Fig. 8: zeigt die Verriegelungsanordnung des Zugspindelaggregats aus Fig. 2 im Längsschnitt im verriegelten Zustand.
- Fig. 9: zeigt die Verriegelungsanordnung des Zugspindelaggregats aus Fig. 2 im Längsschnitt im entriegelten Zustand.

### Detaillierte Beschreibung

In der folgenden Beschreibung werden unter Bezugnahme auf die Zeichnungen beispielhafte Ausführungsformen der vorliegenden Offenbarung beschrieben. Die Zeichnungen sind dabei nicht notwendigerweise maßstabsgetreu, sondern sollen die jeweiligen Merkmale lediglich schematisch illustrieren.

Dabei ist zu beachten, dass die nachstehend beschriebenen Merkmale und Komponenten jeweils miteinander kombiniert werden können, unabhängig davon, ob sie in Zusammenhang mit einer einzigen Ausführungsform beschrieben worden sind. Die Kombination von Merkmalen in den jeweiligen Ausführungsformen dient lediglich der Veranschaulichung des grundsätzlichen Aufbaus und der Funktionsweise der beanspruchten Vorrichtung.

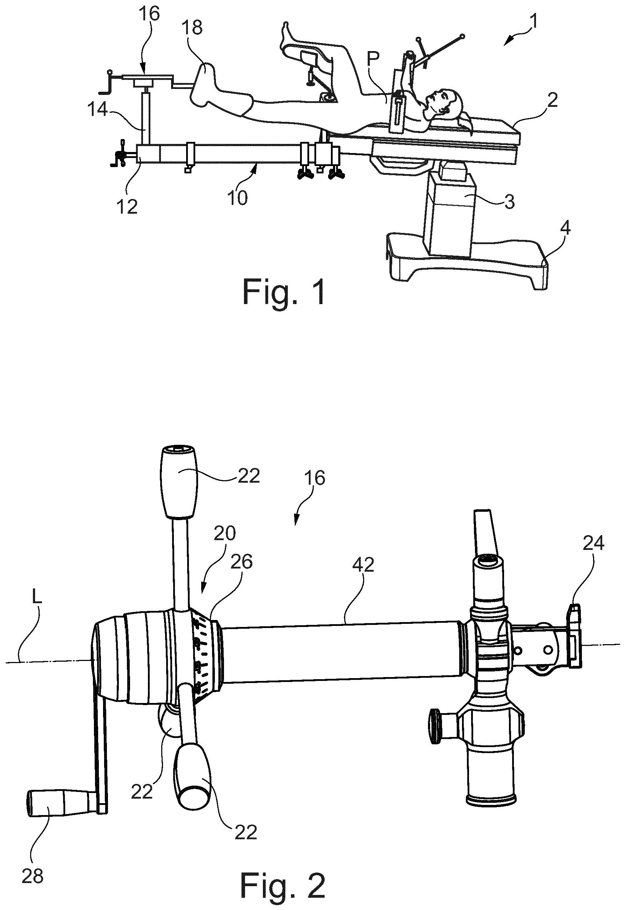

Wie in Fig. 1 dargestellt, umfasst ein Operationstisch 1 eine Patientenlagerfläche 2, eine Säule 3 und einen Fuß 4. Zur Durchführung von orthopädischen Operationen kann ein Extensionsholm 10 verwendet werden, mittels dessen das Bein eines Patienten P in einer gewünschten Position fixiert werden kann. Hierzu kann an einem Holm 12 des Extensionsholms 10 eine verschiebbare Strebe 14 angebracht sein, die wiederum ein Zugspindelaggregat 16 trägt, das mit einer Halterung 18 für einen Patientenfuß verbunden ist. Wenn die verschiebbare Strebe 14 in einer geeigneten Position am Holm 12 fixiert ist, kann somit über das Zugspindelaggregat 16 Zug auf den Patientenfuß in einer Richtung entlang der Längsachse L des Zugspindelaggregats 16 ausgeübt werden, um beispielsweise ein Hüftgelenk des Patienten P auseinander zu ziehen.

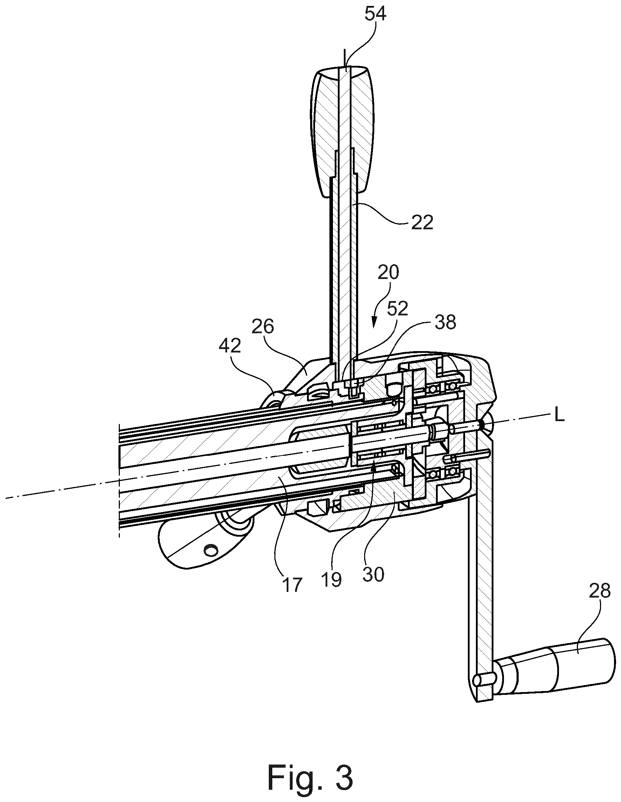

An einem derartigen Zugspindelaggregat 16 kann, wie in Figuren 2 und 3 gezeigt, eine Verriegelungsanordnung 20 gemäß einer Ausführungsform der vorliegenden Offenbarung angebracht sein. Das Zugspindelaggregat 16 umfasst einen Sterngriff 22, mit dem ein Benutzer die Schnittstelle 24 des Zugspindelaggregats 16 um eine Längsachse L des Zugspindelaggregats 16 drehen kann. Somit kann ein in einer Halterung 18 eingespanntes Patientenbein (siehe Fig. 1) mittels des Zugspindelaggregats 16 einerseits durch Betätigen einer Handkurbel 28, welche mit einer Zugspindel 17 eines Spindeltriebs 19 gekoppelt ist, axial entlang der Längsachse L gezogen werden, und andererseits durch Betätigen des Sterngriffs 22 um die Längsachse L gedreht werden. Ein Skalenring 26 in Form einer Schiebehülse zeigt dabei den eingestellten Drehwinkel an. Der Spindeltrieb 19 weist ein Außengewinde auf, welches die Drehung der Handkurbel 28 in eine Längsverschiebung umsetzt.

Die Arme des Sterngriffs 22 erstrecken sich, ausgehend von der Schiebehülse 26, radial nach außen. Die Schiebehülse 26 lässt sich entlang der Längsachse L vor und zurückschieben. Dabei umschließt die Schiebehülse 26 eine Rotor-Statoreinheit 30, 42, auf welcher sie gleitend gelagert ist. Die Rotor-Statoreinheit umfasst einen Rotor ("rotatable bushing") 30 sowie einen Stator ("stationary hub") 42. Der Rotor 30 ist an dem Stator 42 drehbar gelagert. So kann der Rotor 30 sich relativ zum Stator 42 um die Längsachse L drehen.

Wie man es in der Fig. 3 sieht, sind sowohl der Rotor 30 als auch der Stator 42 jeweils als zylindrische Hülse ausgebildet. Diese beiden Hülsen umgeben einen Abschnitt des Spindeltriebs 19. Der Rotor 30 und der Stator 42 sitzen in Richtung der Längsachse L hintereinander. Dabei befindet sich der Stator 42 im Einsatz näher am Patientenbein als der Rotor 30.

Bei der gezeigten Ausführungsform ist der Sterngriff 22 über die Schiebehülse 26 drehfest mit dem Rotor 30 gekoppelt, jedoch in axialer Richtung auf diesem verschieblich. Unter Federlast wird der Sterngriff 22 dabei in seiner vorderen (dem Patienten P zugewandten) Position gehalten, die in den Fig. 2 und 3 gezeigt ist.

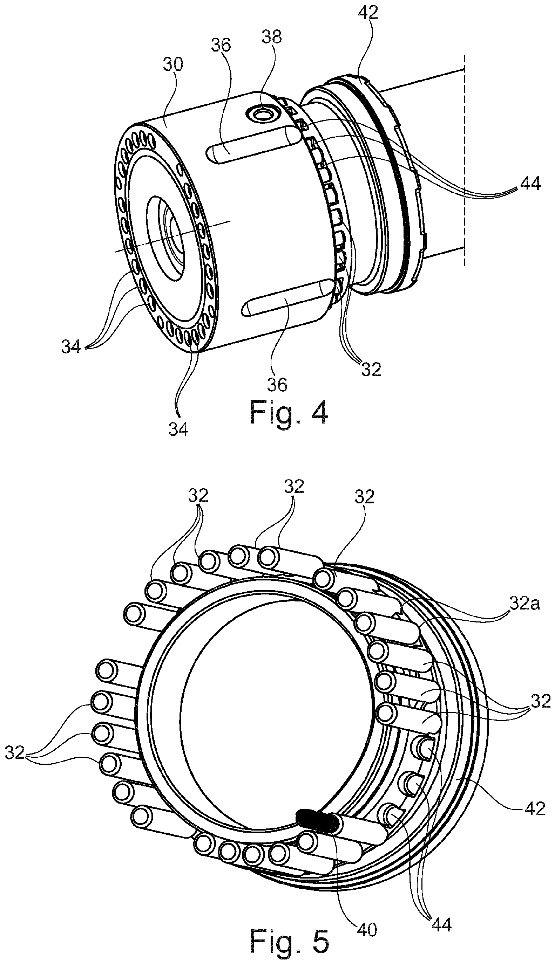

In dieser Stellung ist der Rotor 30 arretiert. Zum Verstellen muss der am Fußende stehende Benutzer den Sterngriff 22 um einige mm zu sich ziehen. Dadurch wird die Arretierung gelöst, wie weiter unten in Zusammenhang mit Fig. 4-6 beschrieben werden wird, und der Rotor 30 kann frei positioniert werden. Lässt der Benutzer den Sterngriff 22 los, bzw. löst den axialen Zug, so bewegt die Federkraft den Sterngriff 22 wieder in Richtung Patient, in die in Fig. 2 gezeigte Grundstellung, und der Rotor 30 wird arretiert. Der Benutzer kann in diesem Fall beide Hände am Sterngriff 22 behalten und kann die Verriegelungsanordnung 20 dabei lösen, den Drehwinkel des Patientenbeins verstellen, und anschließend die Verriegelungsanordnung 20 durch Lösen des axialen Zugs auf den Sterngriff 22 wieder arretieren, ohne den Sterngriff 22 dabei loszulassen. Somit kann der Benutzer das Bein des Patienten exakt positionieren und führen.

Figur 4 zeigt den Rotor 30 der Verriegelungsanordnung 20 aus Fig. 2, wobei eine Mehrzahl von Raststiften 32 in Durchgangsbohrungen 34 im Rotor 30 aufgenommen sind. Ein Stator 42 hat eine Mehrzahl von Rastvertiefungen 44, in welche die Raststifte 32 wahlweise eingreifen können.
Weiterhin sind in Fig. 4 Führungsnuten 36 erkennbar, mittels denen die axiale Bewegung zwischen dem Sterngriff 22 und dem damit verbundenen Skalenring/Schiebehülse 26 einerseits und dem Rotor 30 andererseits geführt werden kann. Eine Rastbuchse 38 am Rotor dient der Arretierung des Sterngriffs 22 in einer entriegelten Stellung der Verriegelungsanordnung 20, wie weiter unten detailliert beschrieben.

Fig. 5 zeigt die im Rotor 30 (siehe Fig. 4) gelagerten Raststifte 32, wobei jeder Raststift 32 über eine zugeordnete Feder 40 in Richtung der Rastvertiefungen 44 im Stator 42 vorgespannt ist. Hierbei ist erkennbar, dass die Anzahl und Verteilung der Raststifte 32 um den Umfang des Rotors 30 herum nicht gleich der Anzahl und Verteilung der Rastvertiefungen 44 am Stator 42 ist. Im gezeigten Beispiel sind die Raststifte 32 in vier Gruppen zu je sechs Raststiften 32 ringförmig um den Umfang des Rotors 30 verteilt, wobei zwischen benachbarten Gruppen von Raststiften 32 ein größerer Abstand ist als zwischen benachbarten Raststiften 32 innerhalb einer Gruppe. Die insgesamt 25 Rastvertiefungen 44 im dargestellten Beispiel sind gleichmäßig um den Umfang des Stators 42 herum verteilt.

Gemäß anderer Ausführungsformen sind jedoch andere Anzahlen an Raststiften 32 und Rastvertiefungen 44 pro Gruppe und pro Rotor bzw. Stator denkbar, solange sichergestellt ist, dass sich die Anzahl und/oder der Winkelabstand der Raststifte 32 von der Anzahl und/oder von dem Winkelabstand der Rastvertiefungen 44 unterscheidet.

So können je nach Anwendungsfall insgesamt 8 bis 60 Raststifte vorgesehen sein. Dabei können 4 bis 10 Stifte zu einer Gruppe zusammengefasst sein. Die Anzahl an Raststiftgruppen kann von 2 bis 6 gehen. Bei den Rastvertiefungen kann die Gesamtanzahl von 9 bis 61 variieren.

Durch die unterschiedliche Anzahl an Raststiften 32 und Rastvertiefungen 44 steht bereits nach Überschreiten eines sehr kleinen Differenzwinkels jeweils ein Raststift 32 aus einer Gruppe genau einer Rastvertiefung 44 gegenüber und wird durch die Federvorspannung in die Rastvertiefung 44 gedrückt. Durch die relativ hohe Anzahl an Raststiften 32 und Rastvertiefungen 44 bei der vorliegenden Ausführungsform, und durch die rotationssymmetrische Anordnung der Gruppen von Raststiften 32, steht dabei in jeder Gruppe genau ein Raststift 32 einer Rastvertiefung 44 gegenüber und greift in diese ein. Durch den gleichzeitigen Eingriff von vier Raststiften 32 bei der dargestellten Ausführungsform können somit durch die Verriegelungsanordnung 20 große Haltekräfte erzielt werden.

Aus der bisherigen Beschreibung ergibt sich, dass die Verriegelungsanordnung 20 die Raststifte 32, die Rastvertiefungen 44 sowie die Schiebehülse 26 umfasst.

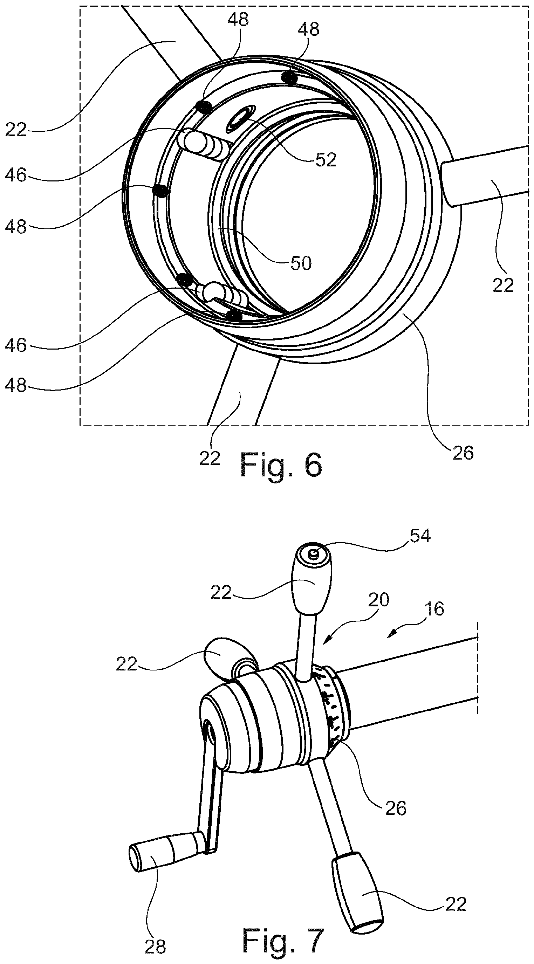

In Fig. 5 ist erkennbar, dass jeweils ein radial außenliegender Abschnitt 32a der Endfläche jedes Raststifts 32 über eine Endfläche der dazugehörigen Rastvertiefung 44 hinausragt. Dieser radial außenliegende Abschnitt 32a kann beim Zurückziehen des Sterngriffs 22 in axialer Richtung von einem Freigabering 50 (siehe Fig. 6) kontaktiert werden, so dass bei axialer Bewegung des Sterngriffs 22 alle Raststifte 32 aus den Rastvertiefungen 44 herausgezogen werden und der Rotor 30 somit frei gegenüber dem Stator 42 drehbar ist.

Fig. 6 zeigt den Sterngriff 22, der an dem Skalenring 26 befestigt ist und zusammen mit diesem in axialer Richtung relativ zum Rotor 30 bewegbar ist. In Führungsnuten 46 am Innenumfang des Skalenrings sind Lagerelemente, wie beispielsweise das gezeigte Kugellager, aufgenommen. Die Lagerelemente greifen in die zugeordneten Führungsnuten 36 am Rotor 30 (siehe Fig. 4) ein. Über Federn 48 ist der Skalenring 26 in Richtung auf eine Verriegelungsstellung der Verriegelungsanordnung 20 vorgespannt, in der jeweils ein Raststift 32 jeder Gruppe in eine Rastvertiefung 44 eingreift.

Am Innenumfang des Skalenrings 26 ist bei der dargestellten Ausführungsform ein umlaufender Vorsprung 50 vorgesehen, welcher als ein Freigabering 50 wirkt und die radial äußeren Abschnitte 32 der Raststifte 32 kontaktiert und bei Bewegung des Sterngriffs 22 in axialer Richtung entlang des Rotors 30 die Raststifte 32 aus den Rastvertiefungen 44 herauszieht. Es ist jedoch auch denkbar, den Freigabering 50 als ein separates Bauteil vorzusehen, das mit dem Sterngriff 22 und/oder dem Skalenring 26 verbunden ist.

Fig. 6 zeigt weiterhin einen Arretierungsstift 52, der in einem der radial ausgerichteten Einzelgriffe des Sterngriffs 22 angeordnet ist, und der in einer zurückgezogenen Stellung des Sterngriffs 22, in der die Raststifte 32 vom Freigabering 50 aus den Rastvertiefungen 44 herausgezogen sind, in die Rastbuchse 38 am Rotor (siehe Fig. 4) eingreifen kann. Damit kann der Sterngriff 22 in einer Freigabestellung der Verriegelungsanordnung 20 arretiert werden. In dieser arretierten Freigabestellung kann der Rotor 30 frei gegenüber dem Stator 42 gedreht werden, ohne dass permanent Zug auf den Sterngriff 22 in axialer Richtung ausgeübt werden muss.

Die Betätigung des Arretierungsstifts 52 kann dabei über einen Druckknopf 54 an einem Ende eines Einzelgriffs des Sterngriffs 22 erfolgen (siehe Fig. 7). Wenn der Benutzer den Sterngriff 22 somit aus der verriegelten Stellung der Verriegelungsanordnung 20 in axialer Richtung auf die Handkurbel 28 zu zieht, und dann in der entriegelten Stellung den Druckknopf 54 betätigt, wird der Sterngriff 22 daran gehindert, wieder in die verriegelte Position zurückzufallen.

Ein erneutes Zurückziehen am Sterngriff 22 gibt den Druckknopf 54 wieder frei und die verriegelte Position, in der jeweils ein Raststift 32 jeder Gruppe von Raststiften in eine Rastvertiefung 44 eingreift und somit die Drehung des Rotors 30 relativ zum Stator 42 blockiert ist, kann wieder eingenommen werden.

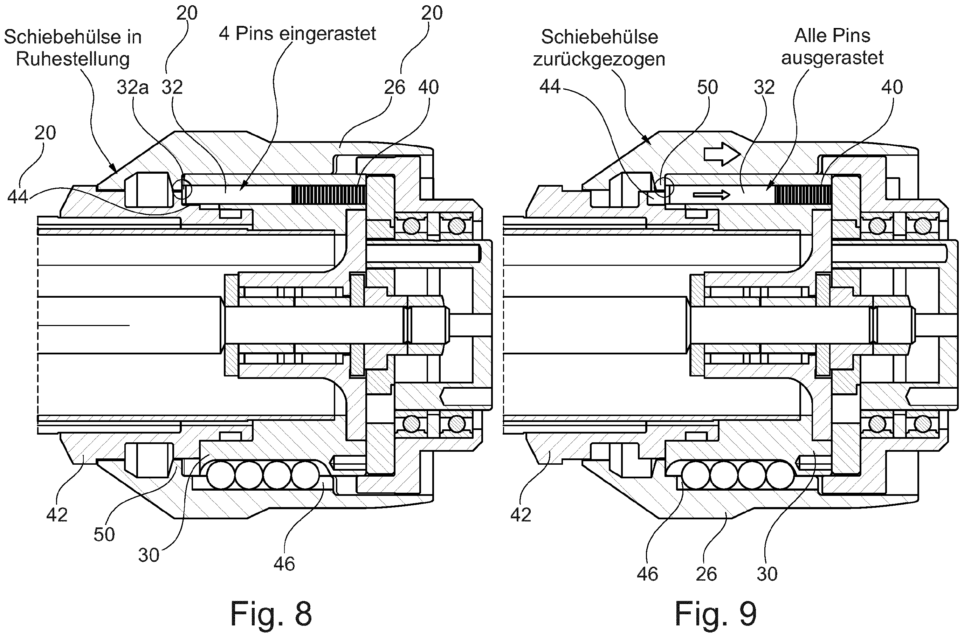

Es wird nun auf die Figuren 8 und 9 eingegangen, welche die Funktionsweise der Verriegelungsanordnung 20 veranschaulichen. Die Figur 8 zeigt den verriegelten Zustand der Verriegelungsanordnung, in welchem der Rotor 30 arretiert ist. Die Figur 9 zeigt den entriegelten Zustand der Verriegelungsanordnung 20, in welchem der Rotor 30 und damit das Patientenbein gedreht werden kann. Die Verriegelung funktioniert wie folgt: mehrere am Umfang des Rotors 30 angeordnete federbelastete Raststifte 32 stehen korrespondierenden Rastvertiefungen 44 am Stator gegenüber. Die Anzahl und Teilung der Vertiefungen 44 und Stifte 32 sind jedoch verschieden. Dadurch stehen sich nie alle Stifte 32 und Vertiefungen 44 gegenüber, jedoch nach Überstreichen des Differenzwinkels steht immer ein Stift 32 einer Gruppe genau gegenüber einer Vertiefung 44. Dadurch lässt sich eine sehr feine Rastierung erreichen, die um ein Vielfaches kleiner ist als die Teilung von Stiften 32 und Vertiefungen 44. Im ausgeführten Beispiel sind die Stifte 32 jeweils zu Gruppen zusammengefasst, wobei sich das Muster viermal am Umfang wiederholt, so dass immer vier Stifte 32 aus den vier verschiedenen Gruppen gleichzeitig einrasten und die Last übertragen.

Die Vertiefungen 44 überdecken den Querschnitt der Raststifte 32 dabei nicht vollständig, sondern der radial äußere Bereich 32a der Stifte steht frei. Der Freigabering 50 nutzt genau diesen äußeren freien Bereich, um die Stifte 32 beim Zurückziehen des Sterngriffes 22 mitzunehmen und damit die Verriegelung zu lösen. Beim Loslassen des Sterngriffes 22 können die Stifte 32 sich wieder auf die Vertiefungen 44 zubewegen und vier Stifte 32 rasten dann davon wieder in gegenüberliegende Vertiefungen 44 ein.

### Bezeichnungen

- 1: Operationstisch
- 2: Patientenlagerfläche
- 3: Säule
- 4: Fuß
- 10: Extensionsholm
- 12: Holm
- 14: verschiebbare Strebe
- 16: Zugspindelaggregat
- 18: Halterung für den Patientenfuß
- 20: Verriegelungsanordnung
- 22: Sterngriff
- 24: Schnittstelle
- 26: Skalenring
- 28: Handkurbel
- 30: Rotor
- 32: Raststift
- 32a: radial äußerer Abschnitt
- 34: Durchgangsbohrung
- 36: Führungsnut
- 38: Rastbuchse
- 40: Feder
- 42: Stator
- 44: Rastvertiefungen
- 46: Führungsnut
- 48: Feder
- 50: Freigabering, Freigabevorsprung
- 52: Arretierungsstift
- L: Längsachse
- P: Patient

## Patentansprüche

1. Rotationsaggregat (16) zur Anbindung an einen Operationstisch (1) zwecks Drehung eines Patientenbeins um seine Längsachse, wobei das Rotationsaggregat (16) folgendes umfasst:
- eine Schnittstelle (24) zur Befestigung des Fußes des Patientenbeins am Rotationsaggregat (16);
- einen Rotor (30), mit dem die Schnittstelle (24) drehfest verbunden ist und der zur Drehung der Schnittstelle (24) und damit des Patientenbeins um die Längsachse dient;
- einen Stator (42), an dem der Rotor (30) drehbar gelagert ist, sodass der Rotor sich relativ zum Stator drehen kann;
- einen Drehgriff (22) zur Drehung des Rotors; und
- eine Verriegelungsanordnung (20) zur Sperrung der Drehung des Rotors relativ zum Stator, **dadurch gekennzeichnet, dass** der Drehgriff (22) verschieblich auf dem Rotor (30) gelagert ist, und
dass die Verriegelungsanordnung (20) durch Verschiebung des Drehgriffs (22) auf dem Rotor betätigt wird.

2. Rotationsaggregat (16) nach Anspruch 1, wobei die Verriegelungsanordnung mindestens zwei Gruppen von Raststiften (32) und eine Mehrzahl von Rastvertiefungen (44) aufweist, wobei die Anzahl der Raststifte (32) verschieden von der Anzahl der Rastvertiefungen (44) ist.

3. Rotationsaggregat (16) nach Anspruch 2, wobei der Rotor (30) am inneren Umfang mehrere Durchgangsbohrungen (34) umfasst, in denen jeweils einer der Raststifte (32) der mindestens zwei Gruppen von Raststiften (32) federnd gelagert und vorgespannt ist.

4. Rotationsaggregat (16) nach Anspruch 2 oder 3, wobei die mindestens zwei Gruppen von Raststiften (32) und die Rastvertiefungen (44) jeweils eine kreisförmige Anordnung bilden.

5. Rotationsaggregat (16) nach einem der Ansprüche 2 bis 4, wobei die mindestens zwei Gruppen von Raststiften (32) und die Rastvertiefungen (44) jeweils radial symmetrisch angeordnet sind.

6. Rotationsaggregat (16) nach einem der Ansprüche 2 bis 5, wobei beim Entriegeln der Verriegelungsanordnung ein gleichzeitiges Herausziehen aller Raststifte (32) aus den Rastvertiefungen (44) gegen die Federvorspannung erfolgt.

7. Rotationsaggregat (16) nach Anspruch 6, wobei das gleichzeitige Herausziehen aller Raststifte (32) aus den Rastvertiefungen (32) durch das Verschieben des Drehgriffs (22) entlang der Drehachse (L) des Rotors (30) erfolgt.

8. Rotationsaggregat (16) nach Anspruch 7, ferner umfassend einen Freigabering (50), der beim Verschieben des Drehgriffs (22) entlang der Drehachse (L) die Raststifte (32) der mindestens zwei Gruppen von Raststiften (32) kontaktiert.

9. Rotationsaggregat (16) nach Anspruch 8, wobei jeweils ein Abschnitt (32a) der Raststifte (32a) radial über die jeweilige Rastvertiefung (44) hervorsteht, und wobei der Freigabering (50) beim Verschieben des Drehgriffs (22) entlang der Drehachse (L) jeweils diesen hervorstehenden Abschnitt (32a) kontaktiert.

10. Rotationsaggregat (16) nach einem der vorherigen Ansprüche, wobei die Verriegelungsanordnung in einer Freigabeposition feststellbar ist, in der der Rotor (30) frei gegenüber dem Stator (42) drehbar ist.

11. Rotationsaggregat (16) nach Anspruch 10, wobei durch das Drücken eines am Drehgriff (22) angebrachten Knopfes (54) die Verriegelungsanordnung in der Freigabeposition feststellbar ist.

12. Rotationsaggregat (16) nach Anspruch 11, wobei der Drehgriff (22) ein Sterngriff (22) mit mehreren Einzelgriffen ist, die sich jeweils in radialer Richtung des Rotors (30) erstrecken, und wobei der Knopf (54) an einem Ende eines Einzelgriffes angeordnet ist.

13. Rotationsaggregat (16) nach einem der Ansprüche 1 bis 12, wobei der Rotor (30) vier Gruppen von Raststiften (32) umfasst, wobei zum Arretieren der Drehbewegung des Rotors (30) jeweils einer der Raststifte (32) der vier Gruppen von Raststiften (32) mit einem Abschnitt vollständig in jeweils einer der Rastvertiefungen (44) aufgenommen ist.

## Claims

1. A rotational unit (16) for connection to an operating table (1) for the purpose of rotating a patient's leg about its longitudinal axis, wherein the rotational unit (16) comprises the following:
- an interface (24) for securing the foot of the patient's leg to the rotational unit (16);
- a rotor (30) to which the interface (24) is connected for conjoint rotation and which serves to rotate the interface (24) and thus the patient's leg about its longitudinal axis;
- a stator (42) on which the rotor (30) is rotatably mounted such that the rotor is able to rotate relative
to the stator;
- a turning handle (22) for rotating the rotor; and
- a locking assembly (20) for blocking the rotation of the rotor relative to the stator, **characterized in that** the turning handle (22) is mounted displaceably on the rotor (30), and
**in that** the locking assembly (20) is actuated by displacing the turning handle (22) on the rotor.

2. The rotational unit (16) according to claim 1, wherein the locking assembly has at least two groups of locking pins (32) and a plurality of locking recesses (44), wherein the number of locking pins (32) is different from the number of locking recesses (44).

3. The rotational unit (16) according to claim 2, wherein the rotor (30) comprises on its inner circumference multiple through bores (34), in which one of the locking pins (32) of the at least two groups of locking pins (32) is respectively spring mounted and preloaded.

4. The rotational unit (16) according to claim 2 or 3, wherein the at least two groups of locking pins (32) and the locking recesses (44) each form a circular arrangement.

5. The rotational unit (16) according to any one of claims 2 to 4, wherein the at least two groups of locking pins (32) and the locking recesses (44) are each arranged radially symmetrically.

6. The rotational unit (16) according to any one of claims 2 to 5, wherein during unlocking of the locking assembly, all the locking pins (32) are pulled simultaneously out of the locking recesses (44) against the spring preload.

7. The rotational unit (16) according to claim 6, wherein the locking pins (32) are all pulled out of the locking recesses (32) simultaneously by displacing the turning handle (22) along the axis of rotation (L) of the rotor (30).

8. The rotational unit (16) according to claim 7, further comprising a release ring (50), which contacts the locking pins (32) of the at least two groups of locking pins (32) when the turning handle (22) is displaced along the axis of rotation (L).

9. The rotational unit (16) according to claim 8, wherein a section (32a) of each of the locking pins (32a) protrudes radially over the respective locking recess (44), and wherein the release ring (50) contacts this protruding section (32a) when the turning handle (22) is displaced along the axis of rotation (L).

10. The rotational unit (16) according to any one of the preceding claims, wherein the locking assembly can be fixed in an unlocked position in which the rotor (30) can rotate freely relative to the stator (42).

11. The rotational unit (16) according to claim 10, wherein the locking assembly can be fixed in the unlocked position by pressing a button (54) mounted on the turning handle (22).

12. The rotational unit (16) according to claim 11, wherein the turning handle (22) is a star handle (22) having multiple individual handles, each extending in the radial direction of the rotor (30), and wherein the button (54) is arranged at one end of an individual handle.

13. The rotational unit (16) according to any one of claims 1 to 12, wherein the rotor (30) comprises four groups of locking pins (32), wherein to arrest the rotational movement of the rotor (30), a section of one of the locking pins (32) in each of the four groups of locking pins (32) is received fully in one of the respective locking recesses (44).

## Revendications

1. Ensemble rotatif (16) destiné à être connecté à une table d'opération (1) dans le but de faire tourner la jambe d'un patient autour de son axe longitudinal, dans lequel l'ensemble rotatif (16) comprend ce qui suit :
- une interface (24) destinée à fixer le pied de la jambe du patient à l'ensemble rotatif (16) ;
- un rotor (30) auquel l'interface (24) est reliée de manière fixe en rotation et qui sert à faire tourner l'interface (24) et donc la jambe du patient autour de l'axe longitudinal ;
- un stator (42) sur lequel le rotor (30) est monté en rotation de sorte que le rotor peut tourner par rapport au stator ;
- une poignée rotative (22) destinée à faire tourner le rotor ; et
- un dispositif de verrouillage (20) destiné à bloquer la rotation du rotor par rapport au stator, **caractérisé en ce que** la poignée rotative (22) est montée de manière coulissante sur le rotor (30), et
**en ce que** le dispositif de verrouillage (20) est actionné par le déplacement de la poignée rotative (22) sur le rotor.

2. Ensemble rotatif (16) selon la revendication 1, dans lequel le dispositif de verrouillage présente au moins deux groupes de broches d'arrêt (32) et une pluralité d'évidements d'arrêt (44), dans lequel le nombre de broches d'arrêt (32) est différent du nombre d'évidements d'arrêt (44).

3. Ensemble rotatif (16) selon la revendication 2, dans lequel le rotor (30) comprend une pluralité d'alésages traversants (34) sur la circonférence intérieure, dans chacun desquels l'une des broches d'arrêt (32) des au moins deux groupes de broches d'arrêt (32) est montée et préchargée par ressort.

4. Ensemble rotatif (16) selon la revendication 2 ou 3, dans lequel les au moins deux groupes de broches d'arrêt (32) et les évidements d'arrêt (44) forment chacun un agencement circulaire.

5. Ensemble rotatif (16) selon l'une des revendications 2 à 4, dans lequel les au moins deux groupes de broches d'arrêt (32) et les évidements d'arrêt (44) sont chacun disposés radialement symétriquement.

6. Ensemble rotatif (16) selon l'une des revendications 2 à 5, dans lequel, lorsque le dispositif de verrouillage est déverrouillé, toutes les broches d'arrêt (32) sont retirées des évidements d'arrêt (44) en même temps contre la précharge du ressort.

7. Ensemble rotatif (16) selon la revendication 6, dans lequel le retrait simultané de toutes les broches d'arrêt (32) des évidements d'arrêt (32) est effectué par le déplacement de la poignée rotative (22) le long de l'axe de rotation (L) du rotor (30) .

8. Ensemble rotatif (16) selon la revendication 7, comprenant en outre une bague de libération (50) qui entre en contact avec les broches d'arrêt (32) des au moins deux groupes de broches d'arrêt (32) lorsque la poignée rotative (22) est déplacée le long de l'axe de rotation (L).

9. Ensemble rotatif (16) selon la revendication 8, dans lequel dans chaque cas une section (32a) des broches d'arrêt (32a) fait saillie radialement au-delà de l'évidement d'arrêt respectif (44), et dans lequel la bague de libération (50) entre en contact avec cette section en saillie (32a) lorsque la poignée rotative (22) est déplacée le long de l'axe de rotation (L).

10. Ensemble rotatif (16) selon l'une des revendications précédentes, dans lequel le dispositif de verrouillage peut être verrouillé dans une position de libération dans laquelle le rotor (30) peut tourner librement par rapport au stator (42).

11. Ensemble rotatif (16) selon la revendication 10, dans lequel le dispositif de verrouillage peut être verrouillé dans la position de libération par l'appui sur un bouton (54) fixé à la poignée rotative (22).

12. Ensemble rotatif (16) selon la revendication 11, dans lequel la poignée rotative (22) est une poignée en étoile (22) comportant plusieurs poignées individuelles, qui s'étendent chacune dans la direction radiale du rotor (30), et dans lequel le bouton (54) est disposé à une extrémité d'une poignée individuelle.

13. Ensemble rotatif (16) selon l'une des revendications 1 à 12, dans lequel le rotor (30) comprend quatre groupes de broches d'arrêt (32), dans lequel, pour verrouiller le mouvement de rotation du rotor (30), l'une respective des broches d'arrêt (32) des quatre groupes de broches d'arrêt (32) est complètement reçue sur une section dans chacun des évidements d'arrêt (44).
